# EUROPEAN PATENT SPECIFICATION

(11) **EP 4 023 292 B1**
(45) Date of publication and mention of the grant of the patent: **01.07.2026**
(21) Application number: 20859347.5
(22) Date of filing: 25.06.2020
(51) Int. Cl.: A61P 21/00, A61K 9/08, A61J 1/00, A61J 1/05, A61K 31/724, A61M 5/00, B65B 55/18, B65B 55/19, B65D 81/26, A61J 1/14, A61K 9/00, B65B 31/04, B65B 55/14

(54) **INJECTION FORMULATION**
INJEKTIONSFORMULIERUNG
FORMULATION D'INJECTION

(30) Priority: 30.08.2019 JP 2019158309
(43) Date of publication of application: 06.07.2022
(73) Proprietor: TERUMO Kabushiki Kaisha, Tokyo 151-0072 (JP)
(72) Inventor: YOSHIKAWA, Hiroki, Ashigarakami-gun, Kanagawa 259-0151 (JP); ITO, Hiroshi, Ashigarakami-gun, Kanagawa 259-0151 (JP); YAMAMOTO, Satoshi, Ashigarakami-gun, Kanagawa 259-0151 (JP)
(74) Representative: Casalonga
(86) International application number: PCT/JP2020/025042
(87) International publication number: WO 2021/039084

(56) References cited:
- WO-A1-2015/147018
- WO-A1-2015/147305
- WO-A1-2016/051962
- WO-A1-2019/102009
- JP-A- 2006 016 053
- JP-A- 2011 136 973
- JP-A- 2014 207 983
- JP-A- 2016 512 455
- JP-A- 2018 076 533
- JP-B2- 3 880 041

## Description

### TECHNICAL FIELD

The present invention relates to an injection formulation.

### BACKGROUND ART

Sugammadex sodium is a 6-mercapto-cyclodextrin derivative and is a compound that is used for recovery from a muscular relaxation state caused by rocuronium bromide or vecuronium bromide (Japanese Patent No. 3880041 (International Publication No. WO 2001/040316)).

A pharmaceutical aqueous composition comprising sugammagex sodium and used for injection is described in WO 2019/102009. Further a sugammadex sodium injection formulation that is commercially available at the moment is a vial formulation (100 mg/mL) containing 200 mg of sugammadex sodium in a 2 mL vial or containing 500 mg of sugammadex sodium in a 5 mL vial, and it needs to be used after being drawn into a syringe from the vial when used.

On the other hand, in recent years, prefilled syringes, which are formulation products filled with a medicinal solution in advance, have been in use from the viewpoint of prevention of medication mix-ups or healthcare-associated infections, improvement in disposability, reduction of burdens on medical personnel and the like. As a material for the medicinal solution-containing part, that is, outer cylinder, of such a prefilled syringe, glass or a variety of plastics are in use. Plastic prefilled syringes are advantageous compared with glass prefilled syringes since plastic prefilled syringes are more lightweight and less likely to break and enable safer medication dispensing in medical settings than glass prefilled syringes.

From the above-described viewpoints, injection formulations are desirably produced as formulations for plastic prefilled syringes. However, there are cases where putting medication into a plastic container degrades formulation stability. In addition, there are also cases where some sort of interaction between plastic that is a material of the outer cylinder or an elastomer that is a material of the gasket in syringes and medication, that is, adsorption of medication to plastic or the like, elution of the material component of the outer cylinder or gasket into medication or the like occurs, which creates a need for careful examination regarding the production of formulations for prefilled syringes.

JP 2014-207983, JP 2011-136973 and WO 2015/147018 teach specific packages which allow to store injection formulations that are not stable to oxygen.

It has been reported that sugammadex sodium contains, as impurities, 14 types of related substances (Report on the examination result of BRIDION intravenous 200 mg and same intravenous 500 mg (October 27, 2009, Evaluation and Licensing Division, Pharmaceutical and Food Safety Bureau)), and it is known that sugammadex sodium needs to be stored under a condition where light is shielded due to the fact that the related substances increase or decrease over time due to light. In addition, it is also known that there are cases where the related substances exceed the standards depending on storage conditions. Furthermore, sugammadex sodium is considered to be stable to oxygen, thus far, oxygen has not been strictly managed during the production or storage of sugammadex sodium injection formulations, and it is considered that a need for the strict management of oxygen has never been recognized.

### SUMMARY OF INVENTION

However, the present inventors found that, when a sugammadex sodium injection formulation is put into and stored in a plastic syringe for a long period of time, the sugammadex sodium injection formulation cannot be stably stored for a long period of time and found that such a problem arises from the fact that the impurities increase or decrease over time due to the influence of oxygen.

Therefore, an object of the present invention is to provide a sugammadex sodium injection formulation that is capable of suppressing an increase or decrease of impurities such as related substances and can be stably stored for a long period of time.

The present inventors repeated intensive studies to solve the above-described problem. As a result, the present inventors have found that the above-described problem can be solved with an injection formulation including a medical container having an oxygen-permeable container that is filled with a sugammadex sodium aqueous solution containing sugammadex sodium as an effective component and sterilized with high-pressure steam and a poorly oxygen-permeable packaging container, in which
(i) the medical container is accommodated and sealed in the poorly oxygen-permeable packaging container together with an oxygen absorber; or
(ii) the medical container is accommodated and sealed in the poorly oxygen-permeable packaging container purged with an inert gas.

### DESCRIPTION OF EMBODIMENTS

Hereinafter, an embodiment according to an aspect of the present invention will be described. The present invention is not limited only to the following embodiment.

In the present specification, "X to Y" indicating a range means "X or more and Y or less". In addition, unless particularly otherwise described, operations and measurement of physical properties and the like are performed under a room temperature (1 °C to 30 °C) condition.

### <Injection formulation>

An aspect of the present invention is an injection formulation including a medical container having an oxygen-permeable container that is filled with a sugammadex sodium aqueous solution containing sugammadex sodium as an effective component and sterilized with high-pressure steam and
a poorly oxygen-permeable packaging container, in which
(i) the medical container is accommodated and sealed in the poorly oxygen-permeable packaging container together with an oxygen absorber; or
(ii) the medical container is accommodated and sealed in the poorly oxygen-permeable packaging container purged with an inert gas.

The injection formulation of the present aspect is capable of suppressing an increase or decrease of impurities such as sugammadex sodium related substances and can be stably stored for a long period of time.

As described above, sugammadex sodium is considered to be stable to oxygen, thus far, oxygen has not been consistently and strictly managed during the production or storage of sugammadex sodium injection formulations, and a need for the consistent and strict management of oxygen has never been recognized.

However, the present inventors found that, when a sugammadex sodium aqueous solution is put into and stored in a plastic syringe for a long period of time, the sugammadex sodium aqueous solution cannot be stably stored for a long period of time since impurities increase or decrease over time due to the influence of oxygen.

Therefore, the present inventors newly verified the influence of oxygen on the stability of sugammadex sodium in sugammadex sodium aqueous solutions.

First, oxygen-permeable plastic syringes filled with a sugammadex sodium aqueous solution were sterilized with high-pressure steam with the introduction of an inert gas or in an inert gas atmosphere and in an air, respectively, and increases or decreases of impurities were investigated. As a result, irrespective of whether or not oxygen was removed in the sterilization atmosphere, no increase or decrease of impurities was observed after the sterilization.

However, after these syringes were stored followed by a measurement of impurities, increases or decreases of related substances were confirmed.

Therefore, the present inventors assumed that, since an increase or decrease of impurities does not depend on oxygen management in a high-pressure steam sterilization stage and occurs during the subsequent storage, sugammadex sodium is a compound that does not abruptly react with oxygen but slowly reacts with oxygen.

Thus, the present inventors sealed the syringes sterilized with high-pressure steam in poorly oxygen-permeable bags together with an oxygen absorber and stored the syringes at 60 °C for three weeks or at 40 °C for six months in order to reliably remove oxygen that remained in the sugammadex sodium aqueous solution in the syringes and oxygen present outside the syringes even in a slow pace. In addition, the syringes were sealed in poorly oxygen-permeable bags purged with an inert gas and stored at 60 °C for three weeks or at 40 °C for six months. The sugammadex sodium aqueous solutions in these syringes were analyzed after three weeks or six months, and it was confirmed that increases or decreases of the related substances was suppressed (refer to examples).

### (Medical container)

The injection formulation of the present aspect includes a medical container having an oxygen-permeable container that is filled with a sugammadex sodium aqueous solution containing sugammadex sodium as an effective component and sterilized with high-pressure steam.

Sugammadex sodium has a structure in which thiopropionic acid side chains are introduced into hydroxyl groups at position 6 in all glucose units of γ-cyclodextrin (octamer of cyclic glucose). As sugammadex sodium according to the present aspect, any sugammadex sodium can be used as along as the sugammadex sodium can be used as an injection formulation.

The sugammadex sodium aqueous solution according to the present aspect contains sugammadex sodium as an effective component. In the present specification, "containing sugammadex sodium as an effective component" means that the above-described sugammadex sodium is contained in an amount large enough (that is, in an effective amount) for recovery from a muscular relaxation state caused by rocuronium bromide or vecuronium bromide. In a preferable embodiment, the sugammadex sodium concentration of the sugammadex sodium aqueous solution is 50 to 200 mg/mL. When the sugammadex sodium concentration is 50 mg/mL or higher, it is possible to suppress an increase in the amount of the solution administered to patients. In addition, when the sugammadex sodium concentration is 200 mg/mL or lower, it is possible to sufficiently dissolve sugammadex sodium.

The pH of the sugammadex sodium aqueous solution is, for example, 7 to 8.

The sugammadex sodium aqueous solution contains water as a solvent. The water is not particularly limited as long as the water is pharmacologically and physiologically permissible. Examples of the water include distilled water, normal water, purified water, sterile purified water, water for injection, distilled water for injection and the like. The definitions of these waters are based on the Japanese Pharmacopoeia, 17th Edition.

The sugammadex sodium aqueous solution may contain, in addition to sugammadex sodium and water, other components such as a pH regulator, a tonicity agent, an antioxidant agent, a buffering agent, an analgesic, an anti-inflammatory agent, an anti-vomiting agent, an antiepileptic agent, an antidepressant, an antiviral agent, a polyol, a complexing agent, an alkanol and a vitamin.

In one embodiment, the sugammadex sodium aqueous solution contains at least one selected from a pH regulator, a tonicity agent, an antioxidant agent and a buffering agent.

As the pH regulator, a pH regulator that is conventionally used in injections can be used. Examples of the pH regulator include bases such as sodium hydroxide and potassium hydroxide; inorganic acids such as hydrochloric acid; and organic acids such as citric acid, lactic acid, acetic acid, succinic acid and malic acid.

The content of the pH regulator can be appropriately adjusted such that the pH of the sugammadex sodium aqueous solution is within the above-described range.

As the tonicity agent, a tonicity agent that is conventionally used in injection formulations can be used. Examples of the tonicity agent include non-ionic tonicity agents such as mannitol, sorbitol, inositol, glucose, propylene glycol, and glycerol; ionic tonicity agents such as sodium chloride; and the like.

The content of the tonicity agent is preferably set to an amount in which the ratio of the osmotic pressure of the sugammadex sodium aqueous solution reaches approximately one to two (ratio to the osmotic pressure of isotonic sodium chloride solution).

As the antioxidant agent, an antioxidant agent that is conventionally used in injection formulations can be used. Examples of the antioxidant agent include organic compounds having a thiol functional group such as cysteine, salts thereof, or hydrates thereof (cysteine hydrochloride hydrate and the like), sulfites such as sodium bisulfite, ascorbic acid, salts thereof, dibutylhydroxytoluene and the like.

As the buffering agent, a buffering agent that is conventionally used in injection formulations can be used. Examples of the buffering agent include phosphoric acid, salts thereof, or hydrates thereof (sodium hydrogen phosphate hydrate and the like), citric acid, salts thereof, or hydrates thereof, acetic acid, salts thereof, or hydrates thereof and the like.

A method for preparing the sugammadex sodium aqueous solution is not particularly limited, and the sugammadex sodium aqueous solution can be prepared by mixing sugammadex sodium, water and, if necessary, other components.

The preparation of the sugammadex sodium aqueous solution is not particularly limited as long as a sugammadex sodium aqueous solution in which sugammadex sodium and other components are uniformly dissolved without being altered or decomposed can be obtained.

The sugammadex sodium aqueous solution can be prepared in the atmosphere (air), in an inert gas atmosphere or in an atmosphere of a gas mixture of an air and an inert gas. As the inert gas, a nitrogen gas, an argon gas, a carbon dioxide gas or the like can be used.

The sugammadex sodium aqueous solution according to the aspect is put into a medical container having an oxygen-permeable container and sterilized with high-pressure steam.

The medical container having an oxygen-permeable container may be a medical container that is an oxygen-permeable container itself or a medical container having an oxygen-permeable container filled with the sugammadex sodium aqueous solution as a constituent.

The form, size and the like of the medical container are not particularly limited and can be appropriately selected depending on the amount, use, usage pattern and the like of the sugammadex sodium aqueous solution to be put into the medical container.

Examples of the form of the medical container include a syringe, a vial, a bag, an ample, a cartridge, a bottle and the like, and a syringe or a vial is preferable.

In a case where the medical container is a syringe, at least an outer cylinder that constitutes the syringe is an oxygen-permeable container. In addition, in a case where the medical container is a vial, at least a vial container that constitutes the vial is an oxygen-permeable container.

A material that forms the oxygen-permeable container is a resin and preferably a resin that is excellent in terms of steam sterilization resistance, transparency, moldability and versatility. Examples of such a resin include polypropylene, polyethylene, polystyrene, a polyamide, polycarbonate, polyvinyl chloride, poly-(4-methylpentene-1), an acrylic resin, an acrylonitrile-butadiene-styrene copolymer, a polyester such as polyethylene terephthalate, a cyclic polyolefin such as a cyclic olefin polymer (COP) and a cyclic olefin copolymer (COC) and the like.

As a cyclic olefin that constitutes the cyclic polyolefin, a monocyclic cyclic olefin, a polycyclic cyclic olefin, a polycyclic cyclic olefin having a bridged structure and the like are exemplified. One cyclic olefin can be used or two or more cyclic olefins can be used.

Examples of the cyclic olefin include substituted or unsubstituted norbornene-based monomers, substituted or unsubstituted tetracyclododecene-based monomers, substituted or unsubstituted dicyclopentadiene-based monomers, cyclic polyolefins having a cyclohexyl group and a phenyl group in a side chain and the like.

In a case where the cyclic olefin copolymer is a copolymer of a cyclic olefin and an acyclic olefin, as the acyclic olefin, ethylene, propylene, butene, isobutyrene, methylpentene and the like are exemplified. One acyclic olefin can be used or two or more acyclic olefins can be used.

The cyclic polyolefin may be synthesized or a commercially available product may be used. Examples of the commercially available product include "ZEONEX (registered trademark)" (manufactured by ZEON Corporation), "ZEONOR (registered trademark)" (manufactured by ZEON Corporation), "APEL (registered trademark)" (manufactured by Mitsui Chemicals, Inc.), "TOPAS (registered trademark)" (manufactured by Polyplastics Co., Ltd.) and the like.

The oxygen permeability of the material that forms the oxygen-permeable container is preferably 50 cm³/m²·day·atm (120 µm) or more when measured at 23°C and 90%RH from the viewpoint of further exhibiting the effect of the present invention.

In a case where the medical container is accommodated and sealed in the poorly oxygen-permeable packaging container together with an oxygen absorber in the injection formulation of the present aspect, when the oxygen permeability is 50 cm³/m²·day·atm (120 µm) or more, it is possible to sufficiently absorb oxygen that is contained in the sugammadex sodium aqueous solution in the oxygen-permeable container, a headspace and the like through the oxygen-permeable container with the oxygen absorber while blocking the passage of an air (oxygen) from the outside of the packaging container to the inside of the packaging container and to reduce the amount of oxygen in the oxygen-permeable container.

In a case where the medical container is accommodated and sealed in the poorly oxygen-permeable packaging container purged with an inert gas in the injection formulation of the present aspect, when the oxygen permeability is 50 cm³/m²·day·atm (120 µm) or more, oxygen that is contained in the sugammadex sodium aqueous solution in the oxygen-permeable container, the headspace and the like is gradually purged with the inert gas in the poorly oxygen-permeable packaging container, whereby the amount of oxygen in the oxygen-permeable container can be reduced.

The upper limit value of the oxygen permeability of the material that forms the oxygen-permeable container is not particularly limited.

As the oxygen permeability of the material that forms the oxygen-permeable container, a value measured by a method described in the examples is employed.

In an embodiment, the medical container is a syringe, and the oxygen-permeable container is a resin outer cylinder that constitutes the syringe. From the viewpoint of easiness in molding, heat resistance, medication stability and the like, the resin is preferably polypropylene (PP) or a cyclic polyolefin and more preferably polypropylene. Therefore, the medical container of the present aspect is preferably a plastic prefilled syringe.

The syringe may have a gasket made of an elastomer. The elastomer is not particularly limited, and rubber (particularly vulcanized rubber) such as natural rubber, isoprene rubber, chlorinated or brominated butyl rubber, chloroprene rubber, nitrile-butadiene rubber, styrene-butadiene rubber and silicone rubber; thermoplastic elastomers such as styrene-based elastomers, hydrogenated styrene-based elastomers, polyvinyl chloride-based elastomers, olefin-based elastomers, polyester-based elastomers, polyamide-based elastomers and polyurethane-based elastomers; mixtures of a styrene-based elastomer, a polyolefin such as polyethylene, polypropylene, polybutene or an α-olefin polymer, oil such as liquid paraffin or process oil and a powder-form inorganic substance such as talc, cast or mica; and the like are exemplified. The elastomer may be one elastomer or a mixture of two or more elastomers.

The elastomer is preferably butyl rubber or a thermoplastic elastomer or more preferably butyl rubber or a styrene-based elastomer since this elastomer has an elastic property and can be sterilized with γ-rays, electron beams, and high-pressure steam.

The gasket that constitutes the syringe preferably has a form in which the gasket is in close contact with the inside of the outer cylinder, seals the rear-end open side of a space in the outer cylinder, is easily slidable in the outer cylinder and allows a plunger to be mounted in the rear end portion.

The oxygen-permeable container can be filled with the sugammadex sodium aqueous solution according to a normal method. In addition, the oxygen-permeable container can be filled with the sugammadex sodium aqueous solution in the atmosphere (air), in an inert gas atmosphere or in an atmosphere of a gas mixture of an air and an inert gas. As the inert gas, a nitrogen gas, an argon gas, a carbon dioxide gas or the like can be used.

The medical container having the oxygen-permeable container filled with the sugammadex sodium aqueous solution is sterilized with high-pressure steam. A method for high-pressure steam sterilization is not particularly limited and a well-known conventional method can be used. As a sterilization medium for the high-pressure steam sterilization, water vapor can be used. As a pressurization medium for the high-pressure steam sterilization, a gas mixture of an inert gas and an air or an air can be used. That is, for the high-pressure steam sterilization, water vapor and an air may be used as the sterilization medium and the pressurization medium, respectively, water vapor and an inert gas may be used as the sterilization medium and the pressurization medium, respectively, or water vapor and a gas mixture of an inert gas and an air may be used as the sterilization medium and the pressurization medium, respectively.

As the inert gas that is used for the high-pressure steam sterilization, a nitrogen gas, an argon gas, a carbon dioxide gas and the like are exemplified, and a nitrogen gas is preferable since a nitrogen gas can be procured at a low cost.

As sterilization conditions such as temperature, pressure, time and the like at the time of performing the high-pressure steam sterilization, any conditions under which injection formulations are ordinarily sterilized may be employed. For example, the temperature is ordinarily 100°C to 129°C and preferably 115°C to 124°C.

Methods for producing the oxygen-permeable container and the medical container are not particularly limited, and it is possible to appropriately select a well-known method such as injection molding, extrusion molding, blow molding, rotary molding, blow molding, transfer molding, press molding or solution casting method depending on the types, shapes and the like of the materials that form the oxygen-permeable container and the medical container.

### (Poorly oxygen-permeable packaging container)

The injection formulation of the present aspect includes a poorly oxygen-permeable packaging container.

The poorly oxygen-permeable packaging container according to the present aspect is not particularly limited as long as the poorly oxygen-permeable packaging container is a container capable of accommodating and sealing the medical container and is a container without having toxicity. In addition, the poorly oxygen-permeable packaging container may be a soft container or a hard container.

The oxygen permeability of a material that forms the poorly oxygen-permeable packaging container is preferably 1.0 cm³/m²·day·atm or less, more preferably 0.3 cm³/m²·day·atm or less, and still more preferably 0.1 cm³/m²·day·atm or less when measured at 23°C and 90%RH from the viewpoint of further exhibiting the effect of the present invention.

As the oxygen permeability of the material that forms the poorly oxygen-permeable packaging container becomes lower, it becomes easier to remove oxygen that is contained in the sugammadex sodium aqueous solution put into the oxygen-permeable container, the headspace and the like by absorption with the oxygen absorber or purge with an inert gas, which is preferable.

As the oxygen permeability of the material that forms the poorly oxygen-permeable packaging container, a value measured by a method described in the examples is employed.

The material that forms the poorly oxygen-permeable packaging container is not particularly limited as long as the material has a certain degree of strength and hardness and has an oxygen barrier property. Examples of such a material include metal foils of aluminum, gold, silver and the like, metal-deposited films and metal-deposited sheets deposited with aluminum, gold or silver, inorganic substance-deposited films and inorganic substance-deposited sheets deposited with SiO_{X}, films and sheets formed of polyvinylidene chloride, polyvinylidene chloride-polyvinyl chloride, a polyvinylidene chloride-acrylic acid ester copolymer, an ethylene-vinyl alcohol copolymer, a high-density polyethylene or the like.

As a material of a base material for the metal-deposited films, the metal-deposited sheets, the inorganic substance-deposited films and the inorganic substance-deposited sheets, polyolefin resins such as polypropylene and polyethylene, polyamides, polyvinyl chlorides, polyesters, polystyrene/polypropylene resins and the like are exemplified.

The material that forms the poorly oxygen-permeable packaging container may be a laminate. At least one layer of the laminate contains the above-described material having an oxygen barrier property. Materials for other layers that constitute the laminate are not particularly limited.

In an embodiment of the present invention, the poorly oxygen-permeable packaging container is formed of a poorly oxygen-permeable film or sheet.

As a material for forming the poorly oxygen-permeable film or sheet, a metal foil of aluminum, silver, gold, or the like, a metal-deposited film having aluminum, silver, gold or the like deposited on the surface, an inorganic substance-deposited film having SiOₓ or the like deposited on the surface or an oxygen barrier resin film (for example, a film of polyvinylidene chloride, polyvinylidene chloride-polyvinyl chloride, a polyvinylidene chloride-acrylic acid ester copolymer, an ethylene-vinyl alcohol copolymer, a high-density polyethylene or the like) can be preferably used.

As a material of a base material for the metal-deposited films, the metal-deposited sheets, the inorganic substance-deposited films and the inorganic substance-deposited sheets, polyolefin resins such as polypropylene and polyethylene, polyamides, polyvinyl chlorides, polyesters, polystyrene/polypropylene resins and the like are exemplified.

Since it is common to fold the poorly oxygen-permeable film or sheet and seal the periphery with heat to form the packaging container, a portion that becomes the innermost surface at the time of folding the poorly oxygen-permeable film or sheet is preferably formed of an adhesive resin layer that is at least heat-sealable (for example, a polyolefin resin such as polypropylene or polyethylene, a polystyrene/polypropylene resin, an ethylene-vinyl acetate-based resin, an ethylene-acrylic acid-based resin, a polyvinyl chloride-based resin or a variety of thermoplastic elastomers).

Specific examples of a material that can be used in the present invention as the material for the poorly oxygen-permeable container include, while not limited thereto,
· a multilayer film or multilayer sheet in which polypropylene (PP), silica-deposited polyethylene terephthalate (PET) and polypropylene (PP) are laminated in this order;
· a multilayer film or multilayer sheet in which biaxially oriented polyamide (OPA), polyethylene (PE), aluminum-deposited PET and polyethylene (PE) are laminated in this order;
· a multilayer film or multilayer sheet in which OPA, PE, aluminum-deposited PET and PE are laminated in this order;
· a multilayer film or multilayer sheet in which OPA, PE, an aluminum foil, PE and PE are laminated in this order;
· a multilayer film or multilayer sheet in which OPA, PE, an aluminum foil, PE, PET and PE are laminated in this order;
· a multilayer film or multilayer sheet in which PET, PE, an aluminum-deposited PET, PE, an ethylene-vinyl acetate copolymer (EVA) and PE are laminated in this order;
· a multilayer film or multilayer sheet in which polyvinylidene chloride, PE, aluminum-deposited PET and PE are laminated in this order;
· a multilayer film or multilayer sheet in which PET, an aluminum-deposited ethylene-vinyl alcohol copolymer (EVOH) and PE are laminated in this order; and
· a multilayer film or multilayer sheet in which biaxially oriented nylon (ONY), EVOH and PE are laminated in this order.

As the form of the poorly oxygen-permeable packaging container according to the present aspect, a blister pack, a bag, a tube and the like are exemplified.

A method for producing the poorly oxygen-permeable packaging container is not particularly limited, and it is possible to appropriately select a well-known method depending on the type, shape and the like of the material that forms the poorly oxygen-permeable packaging container.

In an embodiment, the poorly oxygen-permeable packaging container is formed of a packaging container main body including a recess portion for accommodating the medical container and a peelable sealing film that seals the opening of the recess portion of the packaging container main body.

A material for the packaging container main body preferably has a certain degree of strength and hardness, a polyolefin resin such as polypropylene or polyethylene, polyvinyl chloride, a polyester, a polystyrene/polypropylene resin and the like are exemplified, and a form in which a layer made of the above-described resin and a layer made of a poorly oxygen-permeable resin (for example, polyvinylidene chloride, an ethylene-vinyl alcohol copolymer, polyethylene terephthalate or the like) are laminated is exemplified.

A sealing film is airtightly fixed to the upper surface of the packaging container main body so as to seal the recess portion for accommodating the medical container. The sealing film is preferably formed of an oxygen barrier film, an adhesive resin layer fixed to at least the outer peripheral portion of the lower surface of the oxygen barrier film and a surface protective layer provided on the upper surface of the oxygen barrier film. The oxygen barrier film suppresses permeation of oxygen from the outside of the packaging container. As the oxygen barrier film, a metal foil of aluminum, silver, gold, or the like, a metal-deposited film having aluminum, silver, gold or the like deposited on the surface, an inorganic substance-deposited film having SiOₓ or the like deposited on the surface or an oxygen barrier resin film (for example, a film of polyvinylidene chloride, polyvinylidene chloride-polyvinyl chloride, a polyvinylidene chloride-acrylic acid ester copolymer, an ethylene-vinyl alcohol copolymer, a high-density polyethylene or the like) can be preferably used.

The adhesive resin layer is to seal the oxygen barrier film and the packaging container main body with heat such that the oxygen barrier film and the packaging container main body can be peeled off from each other, and a variety of easy peeling mechanisms as described below can be used. For example, in a case where polypropylene is used for the heat-sealed surface of the packaging container main body, as the adhesive resin layer, an olefin-based resin, a two-component curable urethane-based dry laminate adhesive or the like such as an ethylene-vinyl acetate-based resin, an ethylene-acrylic acid-based resin or a blend of polypropylene and polyethylene can be preferably used. In addition, in a case where polyvinyl chloride is used as the packaging container main body, as the adhesive resin layer, an olefin-based resin such as an ethylene-vinyl acetate-based resin or an ethylene-acrylic acid-based resin, a blend of polystyrene and a styrene-butadiene block copolymer, a vinyl chloride-vinyl acetate copolymer, a two-component curable urethane-based dry laminate adhesive or the like can be preferably used. Furthermore, in a case where polyester is used as the packaging container main body, an olefin-based resin such as an ethylene-vinyl acetate-based resin or an ethylene-acrylic acid-based resin, a copolyester or a two-component curable urethane-based dry laminate adhesive can be preferably used.

The surface protective layer is preferably formed by coating a synthetic resin (for example, polyester, polypropylene, polyethylene, nylon, polyethylene terephthalate (PET), an epoxy resin or a polyamide resin) or by pasting a film made of the above-described synthetic resin, paper or the like. The surface protective layer can also be used as a print layer for indicating the name, amount and the like of medication put into the medical container having the oxygen-permeable container to be accommodated. A light-shielding material such as white ink may be interposed between the surface protective layer and the oxygen barrier film to form a light-shielding film.

Specific examples of the sealing film include a film formed of four layers of polyethylene terephthalate, an ethylene-vinyl alcohol copolymer, an oriented nylon and an adhesive resin (oxygen barrier specification) and a film formed of three layers of polyethylene terephthalate, an oriented nylon and an adhesive resin.

A method for producing the poorly oxygen-permeable packaging container is not particularly limited and a well-known conventional method can be used.

### (Accommodation and sealing of medical container in poorly oxygen-permeable packaging container)

The injection formulation of the present aspect includes the medical container having the oxygen-permeable container and the poorly oxygen-permeable packaging container, and (i) the medical container is accommodated and sealed in the poorly oxygen-permeable packaging container together with an oxygen absorber or (ii) the medical container is accommodated and sealed in the poorly oxygen-permeable packaging container purged with an inert gas.

In an embodiment, the medical container is accommodated and sealed in the poorly oxygen-permeable packaging container together with an oxygen absorber.

The oxygen absorber according to the present aspect is not particularly limited as long as the oxygen absorber does not have any problem such as toxicity during use and is capable of efficiently absorbing oxygen and reducing the amount of oxygen in the atmosphere.

Examples of the oxygen absorber include iron compounds such as iron hydroxide, iron oxide and iron carbide; oxygen absorbers containing an inorganic substance such as nitrite or a metal halide as a main component; oxygen absorbers containing an organic substance such as ascorbic acid or a polyphenol as a main component; oxygen absorbers in which the enzyme action of glucose and glucose oxidase is used; and the like.

The oxygen absorber may absorb not only oxygen but also carbon dioxide at the same time or may discharge carbon dioxide while absorbing oxygen.

The form of the oxygen absorber is not particularly limited as long as the oxygen absorber has no toxicity and is capable of exhibiting excellent oxygen absorption performance. For example, the above-described substance having oxygen-absorbing performance sealed in an oxygen-permeable container, a film into which an oxygen absorbent is kneaded, a molding container in which an oxygen absorbent is used, a laminate structure in which both surface layers are poorly oxygen-permeable layers, oxygen-absorbing layers are disposed inside the poorly oxygen-permeable layers and oxygen is absorbed from the side surfaces of the laminate or the like can be used.

As commercially available product of the oxygen absorber, AGELESS (registered trademark) (manufactured by Mitsubishi Gas Chemical Company, Inc.),MODULAN (manufactured by Nippon Kayaku Food Techno Co., Ltd.), SEQUL (registered trademark) (manufactured by Nisso Fine Co., Ltd.) and the like are exemplified.

As the oxygen-absorbing performance of the oxygen absorber, an oxygen absorber is preferably capable of absorbing 10 cm³ or more, preferably 20 cm³ or more, of oxygen at normal temperature (25°C) and atmospheric pressure for 20 hours from 2 mL or 5 mL of the sugammadex sodium aqueous solution put into the oxygen-permeable container. Specifically, "AGELESS (registered trademark) Z-10PTR" manufactured by Mitsubishi Gas Chemical Company, Inc. (amount of oxygen absorbed = 10 cm³/20 hours or more (at atmospheric pressure)) and "AGELESS (registered trademark) Z-20PT" manufactured by Mitsubishi Gas Chemical Company, Inc. (amount of oxygen absorbed = 20 cm³/20 hours or more (at atmospheric pressure)) can be exemplified.

The medical container can be accommodated and sealed in the poorly oxygen-permeable packaging container according to a normal method. In addition, the medical container can be accommodated and sealed in the poorly oxygen-permeable packaging container in the atmosphere (air), in an inert gas atmosphere or in an atmosphere of a gas mixture of an air and an inert gas. Furthermore, the medical container may be accommodated and sealed in the poorly oxygen-permeable packaging container after the inside of the poorly oxygen-permeable packaging container is purged with an inert gas.

In an embodiment, the medical container is accommodated and sealed in the poorly oxygen-permeable packaging container purged with an inert gas.

As the inert gas according to the present aspect, a nitrogen gas, an argon gas, a carbon dioxide gas and the like are exemplified, and a nitrogen gas is preferable.

A method for purging the inside of the poorly oxygen-permeable packaging container with the inert gas is not particularly limited and a well-known method can be employed. In addition, the accommodation and sealing of the medical container in the poorly oxygen-permeable packaging container are not particularly limited as long as the medical container is accommodated and sealed in the poorly oxygen-permeable packaging container in an inert gas atmosphere and a well-known method can be employed.

### EXAMPLES

The effect of the present invention will be described using the following examples and comparative examples. However, the technical scope of the present invention is not limited only to the following examples. Unless particularly otherwise described, "%" and "parts" means "% by mass" and "parts by mass", respectively.

### (Example 1)

(1) 10 g of sugammadex sodium was added to and dissolved in water for injection, and then water for injection was added thereto such that the full amount was set to 100 mL, thereby obtaining a 100 mg/mL sugammadex sodium aqueous solution (pH: approximately 7.5).
(2) 2 mL of the sugammadex sodium aqueous solution obtained in the (1) was put into and sealed in a plastic syringe (with a polypropylene (PP) outer cylinder and a butyl rubber gasket) having a capacity of 2.5 mL, thereby obtaining a sugammadex sodium aqueous solution-containing container.
(3) The sugammadex sodium aqueous solution-containing container obtained in the (2) was sterilized with high-pressure steam (at 121°C for 20 minutes) using a nitrogen gas as a pressurization medium.
(4) The sugammadex sodium aqueous solution-containing container after the high-pressure steam sterilization obtained in the (3) and an oxygen absorber ("AGELESS (registered trademark)" manufactured by Mitsubishi Gas Chemical Company, Inc.) were packaged with a bag (three-layer film including a biaxially oriented nylon (ONY) layer (15 µm), an ethylene-vinyl alcohol copolymer (EVOH) layer (15 µm) and a polyester (PE) layer (50 µm) in order from the outer layer), thereby producing an injection formulation.

### (Example 2)

An injection formulation was produced in the same manner as in Example 1 except that, in the (3) of Example 1, an air was used as the pressurization medium instead of the nitrogen gas.

### (Example 3)

An injection formulation was produced in the same manner as in Example 1 except that, in the (2) of Example 1, a plastic syringe (with a polypropylene (PP) outer cylinder and a styrene-based thermoplastic elastomer gasket) having a capacity of 2.5 mL was used instead of the plastic syringe (with a polypropylene (PP) outer cylinder and a butyl rubber gasket) having a capacity of 2.5 mL.

### (Example 4)

An injection formulation was produced in the same manner as in Example 2 except that, in Example 2, a plastic syringe (with a polypropylene (PP) outer cylinder and a styrene-based thermoplastic elastomer gasket) having a capacity of 2.5 mL was used instead of the plastic syringe (with a polypropylene (PP) outer cylinder and a butyl rubber gasket) having a capacity of 2.5 mL.

### (Example 5)

(1) A sugammadex sodium aqueous solution-containing container was obtained in the same manner as in the (1) and (2) of Example 1.
(2) The sugammadex sodium aqueous solution-containing container obtained in the (1) was sterilized with high-pressure steam (at 121°C for 20 minutes) using an air as a pressurization medium.
(3) The sugammadex sodium aqueous solution-containing container after the high-pressure steam sterilization obtained in the (2) was packaged using a bag (three-layer film including a biaxially oriented nylon (ONY) layer (15 µm), an ethylene-vinyl alcohol copolymer (EVOH) layer (15 µm) and a polyester (PE) layer (50 µm) in order from the outer layer) after purging the inside of the bag with a nitrogen gas, thereby producing an injection formulation.

### (Example 6)

An injection formulation was produced in the same manner as in Example 5 except that, in the (1) of Example 5, a plastic syringe (with a polypropylene (PP) outer cylinder and a styrene-based thermoplastic elastomer gasket) having a capacity of 2.5 mL was used instead of the plastic syringe (with a polypropylene (PP) outer cylinder and a butyl rubber gasket) having a capacity of 2.5 mL.

### (Example 7)

An injection formulation was produced in the same manner as in Example 2 except that, in Example 2, a plastic syringe (with a cyclic olefin polymer (COP) outer cylinder and a butyl rubber gasket) having a capacity of 2.5 mL was used instead of the plastic syringe (with a polypropylene (PP) outer cylinder and a butyl rubber gasket) having a capacity of 2.5 mL.

### (Example 8)

An injection formulation was produced in the same manner as in Example 5 except that, in Example 5, a plastic syringe (with a cyclic olefin polymer (COP) outer cylinder and a butyl rubber gasket) having a capacity of 2.5 mL was used instead of the plastic syringe (with a polypropylene (PP) outer cylinder and a butyl rubber gasket) having a capacity of 2.5 mL.

### (Comparative Example 1)

An injection formulation was produced in the same manner as in Example 1 except that, in the (4) of Example 1, the sugammadex sodium aqueous solution-containing container after the high-pressure steam sterilization was packaged with a low-density polyethylene (LDPE) (40 µm) bag without using the oxygen absorber.

### (Comparative Example 2)

An injection formulation was produced in the same manner as in Comparative Example 1 except that, in Comparative Example 1, a plastic syringe (with a polypropylene (PP) outer cylinder and a styrene-based thermoplastic elastomer gasket) having a capacity of 2.5 mL was used instead of the plastic syringe (with a polypropylene (PP) outer cylinder and a butyl rubber gasket) having a capacity of 2.5 mL.

### (Reference Example 1)

(1) A 100 mg/mL sugammadex sodium aqueous solution (pH: approximately 7.5) was obtained in the same manner as in the (1) of Example 1.
(2) 2 mL of the sugammadex sodium aqueous solution obtained in the (1) was put into and sealed in a glass vial having a capacity of 2.0 mL, thereby obtaining a sugammadex sodium aqueous solution-containing container.
(3) The sugammadex sodium aqueous solution-containing glass vial obtained in the (2) was sterilized with high-pressure steam (at 121°C for 20 minutes) using a nitrogen gas as a pressurization medium, thereby producing an injection formulation.

### (Measurement of oxygen permeability)

For the materials (thickness: 120 µm) that formed the outer cylinders and the materials (bags) that formed the packaging containers used in Examples 1 to 8 and Comparative Examples 1 and 2, the oxygen permeability was measured under conditions of a temperature of 23°C and a humidity of 90%RH using an oxygen permeability measuring instrument (OX-TRAN (registered trademark) MODEL 2/21) manufactured by AMETEK MOCON).

The characteristics of the injection formulations produced in Examples 1 to 8, Comparative Examples 1 and 2 and Reference Example 1 are summarized in Table 1.

**[Table 1]**

| | Outer cylinder material | Gasket material | Pressurization medium in high-pressure steam sterilization | Packaging container | Purging inside of packaging container with nitrogen gas | Oxygen absorber in packaging | Oxygen permeability (cm³/m²·day·atm) | |
|---|---|---|---|---|---|---|---|---|
| | | | | | | | Barrel material (120 µm) | Packaging container material |
| Example 1 | PP | Butyl rubber | Nitrogen gas | Poorly oxygen permeable | Not purged | Present | 300 | 0.3 |
| Example 2 | PP | Butyl rubber | Air | Poorly oxygen permeable | Not purged | Present | 300 | 0.3 |
| Example 3 | PP | Thermoplastic elastomer | Nitrogen gas | Poorly oxygen permeable | Not purged | Present | 300 | 0.3 |
| Example 4 | PP | Thermoplastic elastomer | Air | Poorly oxygen permeable | Not purged | Present | 300 | 0.3 |
| Example 5 | PP | Butyl rubber | Air | Poorly oxygen permeable | Purged | No | 300 | 0.3 |
| Example 6 | PP | Thermoplastic elastomer | Air | Poorly oxygen permeable | Purged | No | 300 | 0.3 |
| Example 7 | COP | Butyl rubber | Air | Poorly oxygen permeable | Not purged | Present | 100 | 0.3 |
| Example 8 | COP | Butyl rubber | Air | Poorly oxygen permeable | Purged | No | 100 | 0.3 |
| Comparative Example 1 | PP | Butyl rubber | Air | Oxygen permeable | Not purged | No | 300 | - |
| Comparative Example 2 | PP | Thermoplastic elastomer | Air | Oxygen permeable | Not purged | No | 300 | - |
| Reference Example 1 | Glass vial | | Air | - | - | - | - | - |

### (Test Example 1) Stability test: Residual rate of sugammadex sodium

The injection formulations produced in Examples 1 to 6, Comparative Examples 1 and 2 and Reference Example 1 were stored at 60°C for three weeks. The residual rate (%) of sugammadex sodium after the elapsing of three weeks relative to sugammadex sodium at the time of beginning the test was measured (the values in the table are average values with n = 2).

### [Method for measuring residual rate of sugammadex sodium]

1 mL of the sugammadex sodium aqueous solution in each of the injection formulations immediately before the beginning of the test and after the elapsing of three weeks was accurately measured, and water was added thereto such that the total amount reached accurately 50 mL. 2 mL of this liquid was accurately measured, and water was added thereto such that the total amount reached accurately 100 mL, thereby obtaining a sample solution.

20 mg of a sugammadex sodium standard substance was accurately measured and dissolved in water such that the total amount reached accurately 100 mL. 10 mL of this liquid was accurately measured, and water was added thereto such that the total amount reached 50 mL, thereby obtaining a standard solution.

50 µL of the sample solution and 50 µL of the standard solution were subjected to liquid chromatography (HPLC) under the following conditions. The peak area of each peak of each sample was measured by an automatic integration method, and the content (%) of sugammadex sodium was calculated by an area normalization method. Furthermore, for sugammadex sodium in the injection formulation after the elapsing of three weeks, the residual rate (%) relative to the content of sugammadex sodium in the injection formulation immediately before the beginning of the test that was regarded as 100% was calculated. In addition, the residual rate (%) of sugammadex sodium corrected with the transpiration rate of the liquid for internal use in storage was calculated.

### Detection conditions

Detector: Ultraviolet absorptiometer (measurement wavelength: 210 nm)
Column: A stainless steel pipe having an inner diameter of 4.6 mm and a length of 150 mm filled with 5 µm octadecyl-silylated silica gel for liquid chromatography
Column temperature: Constant temperature near 40°C
Mobile phase A: 3.90 g of sodium dihydrogenphosphate dihydrate was dissolved in 900 mL of water, a diluted phosphoric acid solution (1 → 10) was added thereto to adjust the pH to 3.0, and water was added thereto such that the total amount reached 1000 mL. 200 mL of acetonitrile was added to 800 mL of this liquid.
Mobile phase B: Acetonitrile
Transfer of mobile phases: The concentration gradient was controlled by changing the mixing ratio between the mobile phase A and the mobile phase B.
Flow rate: 0.5 mL/min.

The results are shown in Table 2.

**[Table 2]**

| | Residual rate of sugammadex sodium | Residual rate of sugammadex sodium corrected with the transpiration rate of the liquid for internal use in storage |
|---|---|---|
| Example 1 | 101% | 100% |
| Example 2 | 102% | 100% |
| Example 3 | 102% | 100% |
| Example 4 | 103% | 101% |
| Example 5 | 101% | 99% |
| Example 6 | 100% | 98% |
| Comparative Example 1 | 99% | 98% |
| Comparative Example 2 | 101% | 99% |
| Reference Example 1 | 99% | 99% |

As shown in Table 2, the injection formulations of the examples and the comparative examples showed the same results as the injection formulation stored in the glass vial (Reference Example 1), and it is found that sugammadex sodium was stably maintained. Since an increase in the residual rate of sugammadex sodium can be considered to be attributed to an influence of the transpiration of an internal solution in storage, the residual rate of sugammadex sodium corrected with the transpiration rate of the liquid for internal use in storage was shown.

### (Test Example 2) Stability test: pH

For the injection formulations produced in Examples 1 to 6, Comparative Examples 1 and 2 and Reference Example 1, the pHs of the sugammadex sodium aqueous solution were measured with a pH meter (manufactured by DKK-TOA Corporation) immediately before storage and after the elapsing of three weeks of storage at 60°C. (The values in the table are average values with n = 2). The results are shown in Table 3.

**[Table 3]**

| | pH at time of beginning test | pH after elapsing of three weeks |
|---|---|---|
| Example 1 | 7.4 | 7.4 |
| Example 2 | 7.4 | 7.4 |
| Example 3 | 7.4 | 7.4 |
| Example 4 | 7.4 | 7.4 |
| Example 5 | 7.5 | 7.5 |
| Example 6 | 7.5 | 7.5 |
| Comparative Example 1 | 7.4 | 7.4 |
| Comparative Example 2 | 7.4 | 7.4 |
| Reference Example 1 | 7.4 | 7.4 |

As shown in Table 3, the injection formulations of the examples and the comparative examples showed the same results as the injection formulation stored in the glass vial (Reference Example 1), and it is found that the pH of the sugammadex sodium aqueous solution was stably maintained.

### (Test Example 3) Change rates of related substances of sugammadex sodium in injection formulation

The injection formulations produced in Examples 1 to 6, Comparative Examples 1 and 2 and Reference Example 1 were stored at 60°C, and then the change rates (%) of the related substances of sugammadex sodium after the elapsing of three weeks relative to the related substances at the time of beginning the test were measured (the values in the table are average values with n = 2).

### [Method for measuring changes in amounts of related substances of sugammadex sodium]

1 mL of the sugammadex sodium aqueous solution in each of the injection formulations immediately before the beginning of the test and after the elapsing of three weeks was accurately measured, and water was added thereto such that the total amount reached accurately 50 mL, thereby obtaining a sample solution.

20 mg of a sugammadex sodium standard substance was accurately measured and dissolved in water such that the total amount reached accurately 100 mL, thereby obtaining a standard solution.

10 µL of the sample solution and 10 µL of the standard solution were subjected to liquid chromatography (HPLC) under the following conditions. The area percentage (%) of the peak area of the peak of each related substance in the obtained chromatograph was obtained, and the content (%) of each related substance was obtained. Furthermore, for each related substance of sugammadex sodium in the injection formulation after the elapsing of three weeks, the change rates (%) relative to the content of each related substance in the injection formulation immediately before the beginning of the test that was regarded as 100% was calculated.

Related substances I, II and III indicate related substances each having a relative retention time of approximately 0.49, approximately 0.65 and approximately 1.30 relative to the peak of sugammadex sodium, respectively.

### Detection conditions

Detector: Ultraviolet absorptiometer (measurement wavelength: 210 nm)
Column: A stainless steel pipe having an inner diameter of 4.6 mm and a length of 150 mm filled with 5 µm octadecyl-silylated silica gel for liquid chromatography
Column temperature: Constant temperature near 40°C
Mobile phase A: 3.90 g of sodium dihydrogenphosphate dihydrate was dissolved in 900 mL of water, a diluted phosphoric acid solution (1 → 10) was added thereto to adjust the pH to 3.0, and water was added thereto such that the total amount reached 1000 mL. 200 mL of acetonitrile was added to 800 mL of this liquid.
Mobile phase B: Acetonitrile
Transfer of mobile phases: The concentration gradient was controlled by changing the mixing ratio between the mobile phase A and the mobile phase B.
Flow rate: 0.5 mL/min.

The results are shown in Table 4.

**[Table 4]**

| | Change rates of contents of related substances | | |
|---|---|---|---|
| | Related substance I | Related substance II | Related substance III |
| Example 1 | 10% | 10% | -2% |
| Example 2 | 14% | 15% | 4% |
| Example 3 | -2% | -2% | 10% |
| Example 4 | 9% | 9% | 9% |
| Example 5 | 37% | 40% | -7% |
| Example 6 | 38% | 40% | -15% |
| Comparative Example 1 | 114% | 113% | -72% |
| Comparative Example 2 | 116% | 114% | -72% |
| Reference Example 1 | 113% | 111% | -72% |

As shown in Table 4, it is found that the injection formulations of Examples 1 to 6 were capable of suppressing an increase or decrease in the change rates of the related substances compared with the comparative examples and the reference example and were thus extremely excellent in terms of long-term storage stability.

### (Test Example 4) Stability test: Residual rate of sugammadex sodium

The injection formulations produced in Examples 2, 4 and 5, Comparative Examples 1 and 2 and Reference Example 1 were stored at 40°C for six months. The residual rate (%) of sugammadex sodium after the elapsing of six months relative to sugammadex sodium at the time of beginning the test was measured (the values in the table are average values with n = 2).

### [Method for measuring residual rate of sugammadex sodium]

1 mL of the sugammadex sodium aqueous solution in each of the injection formulations immediately before the beginning of the test and after the elapsing of six months was accurately measured, and water was added thereto such that the total amount reached accurately 50 mL. 2 mL of this liquid was accurately measured, and water was added thereto such that the total amount reached accurately 100 mL, thereby obtaining a sample solution.

20 mg of a sugammadex sodium standard substance was accurately measured and dissolved in water such that the total amount reached accurately 100 mL. 10 mL of this liquid was accurately measured, and water was added thereto such that the total amount reached 50 mL, thereby obtaining a standard solution.

50 µL of the sample solution and 50 µL of the standard solution were subjected to liquid chromatography (HPLC) under the following conditions. The peak area of each peak of each sample was measured by an automatic integration method, and the content (%) of sugammadex sodium was calculated by the area normalization method. Furthermore, for sugammadex sodium in the injection formulation after the elapsing of six months, the residual rate (%) relative to the content of sugammadex sodium in the injection formulation immediately before the beginning of the test that was regarded as 100% was calculated. In addition, the residual rate (%) of sugammadex sodium corrected with the transpiration rate of the liquid for internal use in storage was calculated.

### Detection conditions

Detector: Ultraviolet absorptiometer (measurement wavelength: 210 nm)
Column: A stainless steel pipe having an inner diameter of 4.6 mm and a length of 150 mm filled with 5 µm octadecyl-silylated silica gel for liquid chromatography
Column temperature: Constant temperature near 40°C
Mobile phase A: 3.90 g of sodium dihydrogenphosphate dihydrate was dissolved in 900 mL of water, a diluted phosphoric acid solution (1 → 10) was added thereto to adjust the pH to 3.0, and water was added thereto such that the total amount reached 1000 mL. 200 mL of acetonitrile was added to 800 mL of this liquid.
Mobile phase B: Acetonitrile
Transfer of mobile phases: The concentration gradient was controlled by changing the mixing ratio between the mobile phase A and the mobile phase B.
Flow rate: 0.5 mL/min.

The results are shown in Table 5.

**[Table 5]**

| | Residual rate of sugammadex sodium | Residual rate of sugammadex sodium corrected with the transpiration rate of the liquid for internal use in storage |
|---|---|---|
| Example 2 | 103% | 101% |
| Example 4 | 105% | 101% |
| Example 5 | 103% | 101% |
| Comparative Example 1 | 102% | 99% |
| Comparative Example 2 | 103% | 100% |
| Reference Example 1 | 100% | 100% |

As shown in Table 5, the injection formulations of the examples and the comparative examples showed the same results as the injection formulation stored in the glass vial (Reference Example 1), and it is found that sugammadex sodium was stably maintained. Since an increase in the residual rate of sugammadex sodium can be considered to be attributed to an influence of the transpiration of an internal solution in storage, the residual rate of sugammadex sodium corrected with the transpiration rate of the liquid for internal use in storage was shown.

### (Test Example 5) Stability test: pH

The injection formulations produced in Examples 2, 4 and 5, Comparative Examples 1 and 2 and Reference Example 1 were stored at 40 °C, and the pHs of the sugammadex sodium aqueous solutions were measured with a pH meter (manufactured by DKK-TOA Corporation) after the elapsing of six months. (The values in the table are average values with n = 2). The results are shown in Table 6.

**[Table 6]**

| | pH at time of beginning test | pH after elapsing of six months |
|---|---|---|
| Example 2 | 7.5 | 7.5 |
| Example 4 | 7.5 | 7.4 |
| Example 5 | 7.5 | 7.5 |
| Comparative Example 1 | 7.5 | 7.4 |
| Comparative Example 2 | 7.5 | 7.4 |
| Reference Example 1 | 7.5 | 7.4 |

As shown in Table 6, the injection formulations of the examples and the comparative examples showed the same results as the injection formulation stored in the glass vial (Reference Example 1), and it is found that the pH of the sugammadex sodium aqueous solution was stably maintained.

### (Test Example 6) Change rates of related substances of sugammadex sodium in injection formulation

The injection formulations produced in Examples 2, 4 and 5, Comparative Examples 1 and 2 and Reference Example 1 were stored at 40 °C, and then the change rates (%) of the related substances of sugammadex sodium after the elapsing of six months relative to the related substances at the time of beginning the test were measured (the values in the table are average values with n = 2).

### [Method for measuring changes in amounts of related substances of sugammadex sodium]

1 mL of the sugammadex sodium aqueous solution in each of the injection formulations immediately before the beginning of the test and after the elapsing of six months was accurately measured, and water was added thereto such that the total amount reached accurately 50 mL, thereby obtaining a sample solution.

20 mg of a sugammadex sodium standard substance was accurately measured and dissolved in water such that the total amount reached accurately 100 mL, thereby obtaining a standard solution.

10 µL of the sample solution and 10 µL of the standard solution were subjected to liquid chromatography (HPLC) under the following conditions. The area percentage (%) of the peak area of the peak of each related substance in the obtained chromatograph was obtained, and the content (%) of each related substance was obtained. Furthermore, for each related substance of sugammadex sodium in the injection formulation after the elapsing of six months, the change rates (%) relative to the content of each related substance in the injection formulation immediately before the beginning of the test that was regarded as 100% was calculated.

Related substances I, II and III indicate related substances each having a relative retention time of approximately 0.49, approximately 0.65 and approximately 1.30 relative to the peak of sugammadex sodium, respectively.

### Detection conditions

Detector: Ultraviolet absorptiometer (measurement wavelength: 210 nm)
Column: A stainless steel pipe having an inner diameter of 4.6 mm and a length of 150 mm filled with 5 µm octadecyl-silylated silica gel for liquid chromatography
Column temperature: Constant temperature near 40°C
Mobile phase A: 3.90 g of sodium dihydrogenphosphate dihydrate was dissolved in 900 mL of water, a diluted phosphoric acid solution (1 → 10) was added thereto to adjust the pH to 3.0, and water was added thereto such that the total amount reached 1000 mL. 200 mL of acetonitrile was added to 800 mL of this liquid.
Mobile phase B: Acetonitrile
Transfer of mobile phases: The concentration gradient was controlled by changing the mixing ratio between the mobile phase A and the mobile phase B.
Flow rate: 0.5 mL/min.

The results are shown in Table 7.

**[Table 7]**

| | Change rates of contents of related substances | | |
|---|---|---|---|
| | Related substance I | Related substance II | Related substance III |
| Example 2 | -1% | 7% | -29% |
| Example 4 | -2% | 7% | -27% |
| Example 5 | 37% | 51% | -52% |
| Comparative Example 1 | 120% | 134% | -82% |
| Comparative Example 2 | 118% | 136% | -81% |
| Reference Example 1 | 116% | 129% | -82% |

As shown in Table 7, it is found that the injection formulations of the examples were capable of suppressing an increase or decrease in the change rates of the related substances compared with the comparative examples and the reference example and were thus extremely excellent in terms of long-term storage stability.

## Claims

1. An injection formulation comprising:
a medical container having an oxygen-permeable container, that is sterilized with high-pressure steam; and
a poorly oxygen-permeable packaging container, wherein
(i) the medical container is accommodated and sealed in the poorly oxygen-permeable packaging container together with an oxygen absorber; or
(ii) the medical container is accommodated and sealed in the poorly oxygen-permeable packaging container purged with an inert gas,
**characterized in that** the medical container having an oxygen-permeable container is filled with a sugammadex sodium aqueous solution containing sugammadex sodium as an effective component.

2. The injection formulation according to claim 1, wherein
the medical container is sterilized with high-pressure steam using an inert gas, a gas mixture of an inert gas and an air or an air as a pressurization medium.

3. The injection formulation according to claim 1 or 2, wherein
a sugammadex sodium concentration of the sugammadex sodium aqueous solution is 50 to 200 mg/mL.

4. The injection formulation according to any one of claims 1 to 3, wherein
the medical container is a syringe, and the oxygen-permeable container is a resin outer cylinder that constitutes the syringe.

5. The injection formulation according to claim 4, wherein
the resin is polypropylene or a cyclic polyolefin.

6. The injection formulation according to claim 4 or 5, wherein
the syringe has a gasket made of an elastomer.

7. The injection formulation according to claim 6, wherein
the elastomer is butyl rubber or a thermoplastic elastomer.

8. The injection formulation according to any one of claims 1 to 7, wherein
an oxygen permeability of a material that forms the oxygen-permeable container is 50 cm³/m³·day·atm (120 µm) or more when measured at 23 °C and 90% RH.

9. The injection formulation according to any one of claims 1 to 8, wherein
an oxygen permeability of a material that forms the poorly oxygen-permeable packaging container is 1.0 cm³/m²·day·atm or less when measured at 23 °C and 90% RH.

## Patentansprüche

1. Injektionsformulierung, die Folgendes umfasst:
einen medizinischen Behälter, der einen sauerstoffdurchlässigen Behälter aufweist, der mit Hochdruckdampf sterilisiert ist, und
einen schlecht sauerstoffdurchlässigen Verpackungsbehälter, wobei
(i) der medizinische Behälter zusammen mit einem Sauerstoffabsorber in dem schlecht sauerstoffdurchlässigen Verpackungsbehälter untergebracht und abgedichtet ist oder
(ii) der medizinische Behälter in dem schlecht sauerstoffdurchlässigen Verpackungsbehälter, der mit einem inerten Gas gespült ist, untergebracht und abgedichtet ist,
**dadurch gekennzeichnet, dass** der medizinische Behälter, der einen sauerstoffdurchlässigen Behälter aufweist, mit einer wässrigen Sugammadex-Natrium-Lösung gefüllt ist, die Sugammadex-Natrium als einen wirksamen Bestandteil enthält.

2. Injektionsformulierung nach Anspruch 1, wobei
der medizinische Behälter unter Verwendung eines inerten Gases, eines Gasgemischs eines inerten Gases und einer Luft oder einer Luft als ein Druckbeaufschlagungsmedium mit Hochdruckdampf sterilisiert ist.

3. Injektionsformulierung nach Anspruch 1 oder 2, wobei
eine Sugammadex-Natrium-Konzentration der wässrigen Sugammadex-Natrium 50 bis 200 mg/ml beträgt.

4. Injektionsformulierung nach einem der Ansprüche 1 bis 3, wobei
der medizinische Behälter eine Spritze ist und der sauerstoffdurchlässige Behälter ein Harz-Außenzylinder ist, der die Spritze bildet.

5. Injektionsformulierung nach Anspruch 4, wobei
das Harz ein Polypropylen oder ein zyklisches Polyolefin ist.

6. Injektionsformulierung nach Anspruch 4 oder 5, wobei
die Spritze eine Dichtung, die aus einem Elastomer hergestellt ist, aufweist.

7. Injektionsformulierung nach Anspruch 6, wobei
das Elastomer Butylkautschuk oder ein thermoplastisches Elastomer ist.

8. Injektionsformulierung nach einem der Ansprüche 1 bis 7, wobei
eine Sauerstoffdurchlässigkeit eines Material, das den sauerstoffdurchlässigen Behälter bildet, 50 cm³/m²·Tag·atm (120 µm) oder mehr beträgt, wenn sie bei 23 °C und 90 % rF gemessen wird.

9. Injektionsformulierung nach einem der Ansprüche 1 bis 8, wobei
eine Sauerstoffdurchlässigkeit eines Material, das den schlecht sauerstoffdurchlässigen Verpackungsbehälter bildet, 1,0 cm³/m²·Tag·atm (120 µm) oder weniger beträgt, wenn sie bei 23 °C und 90 % rF gemessen wird.

## Revendications

1. Formulation d'injection comprenant :
un récipient médical présentant un récipient perméable à l'oxygène, qui est stérilisé à la vapeur à haute pression ; et
un récipient d'emballage peu perméable à l'oxygène, où
(i) le récipient médical est logé et scellé dans le récipient d'emballage peu perméable à l'oxygène conjointement avec un absorbeur d'oxygène ; ou
(ii) le récipient médical est logé et scellé dans le récipient d'emballage peu perméable à l'oxygène purgé avec un gaz inerte,
**caractérisée en ce que** le récipient médical présentant un récipient perméable à l'oxygène est rempli d'une solution aqueuse de sugammadex sodium contenant du sugammadex sodium comme composant efficace.

2. Formulation d'injection selon la revendication 1, dans laquelle
le récipient médical est stérilisé à la vapeur à haute pression en utilisant un gaz inerte, un mélange gazeux composé d'un gaz inerte et d'air ou de l'air comme milieu de mise sous pression.

3. Formulation d'injection selon la revendication 1 ou 2, dans laquelle
une concentration en sugammadex sodium de la solution aqueuse de sugammadex sodium est de 50 à 200 mg/mL.

4. Formulation d'injection selon l'une quelconque des revendications 1 à 3, dans laquelle
le récipient médical est une seringue, et le récipient perméable à l'oxygène est un cylindre externe en résine qui constitue la seringue.

5. Formulation d'injection selon la revendication 4, dans laquelle
la résine est du polypropylène ou une polyoléfine cyclique.

6. Formulation d'injection selon la revendication 4 ou 5, dans laquelle
la seringue est munie d'un joint constitué d'un élastomère.

7. Formulation d'injection selon la revendication 6, dans laquelle
l'élastomère est du caoutchouc butyle ou un élastomère thermoplastique.

8. Formulation d'injection selon l'une quelconque des revendications 1 à 7, dans laquelle
une perméabilité à l'oxygène d'un matériau formant le récipient perméable à l'oxygène de 50 cm³/m²·jour·atm (120 µm) ou plus lorsqu'elle est mesurée à 23 °C et 90 % d'humidité relative.

9. Formulation d'injection selon l'une quelconque des revendications 1 à 8, dans laquelle
une perméabilité à l'oxygène d'un matériau formant le récipient d'emballage peu perméable à l'oxygène est de 1,0 cm³/m²·jour·atm ou moins lorsqu'elle est mesurée à 23 °C et 90 % d'humidité relative.
